# EUROPEAN PATENT APPLICATION

(11) **EP 1 561 471 A1**
(43) Date of publication of application: **10.08.2005**
(21) Application number: 03751454.4
(22) Date of filing: 10.10.2003
(51) Int. Cl.: A61K 45/00, A61K 31/198, A61K 31/205, A61K 39/395, A61P 35/00, A61P 43/00

(54) **CELL GROWTH REGULATOR**

(30) Priority: 11.10.2002 JP 2002298701
(71) Applicant: CHUGAI SEIYAKU KABUSHIKI KAISHA, Tokyo, 115-8543 (JP)
(72) Inventor: HATANAKA, Takahiro, Chugai Seiyaku KK, Gotenba-shi, Shizuoka 412-8513 (JP); OHIZUMI, Iwao, Chugai Seiyaku Kabushiki Kaisha, Gotenba-shi, Shizuoka 412-8513 (JP); NEZU, Jun-ichi, Chugai Seiyaku Kabushiki Kaisha, Niihari-gun, Ibaraki 300-4101 (JP)
(74) Representative: Bassil, Nicholas Charles
(86) International application number: PCT/JP2003/013061
(87) International publication number: WO 2004/032966

(57) **Abstract**

The present inventors examined cell growth of human colon cancer cell lines and breast cancer cell lines using L-2-phenylglycine, which is considered to be an amino acid transporter ATB^{0,+} inhibitor, as a test substance to reveal a concentration-dependent cell growth inhibitory effect. This result shows that inhibition of ATB^{0,+} activity may suppress cell growth. Suppression of the amino acid transporter ATB^{0,+} activity may be an important index in developing growth inhibitors for cancer cells and such.

## Description

### Technical Field

The present invention relates to cell growth inhibitors comprising an amino acid transporter ATB^{0,+} inhibitory substance as an active ingredient.

### Background Art

Mammals need to take in external sources of nutrition, and many transport proteins are known to exist in cells. Amino acid transporters (amino acid transport proteins) transport amino acids. Many amino acid transporters have been discovered to date (see, Non-Patent Document 1).

Amino acid transporters are reported to be involved in the transport of not only natural amino acids, but also of pharmaceutical agents such as L-α-methyldopa and NOS inhibitors (see Non-Patent Documents 2 and 3).

ATB^{0,+} is an amino acid transporter expressed in the intestines, lungs, and mammary glands. Functionally, ATB^{0,+} is a Na⁺- and Cl⁻-driven neutral and cationic amino acid transport system. This system plays an important role in the absorption of amino acids in the intestinal tract (see, Non-Patent Document 1). Cloning of human ATB^{0,+} has been recently reported (see, Non-Patent Document 4). To date, the transport function of ATB^{0,+} has only been studied for amino acids as substrates. Their transport function has a strong concentrating power, and is energized by transmembrane Na⁺ and Cl⁻ gradients, as well as by membrane potential. ATB^{0,+} belongs to the genetic family of Na⁺- and Cl⁻-driven transporters for a variety of compounds such as amino acids (e.g., glycine and proline), neurotransmitters (e.g., monoamines and GABA), and osmolytes (e.g., taurine and betaine). Structurally, ATB^{0,+} is related very closely to GABA transporters and betaine transporters.

ATB^{0,+} is a 12-transmembrane protein comprising 642 amino acids (Non-Patent Literature 4). Its homology between humans and mice at the amino acid level is 88%, and homology at the extracellular regions of TM3-4 is 77%. The physiological functions of ATB^{0,+} are considered to be involved in the efficient absorption of nutritional substances in the digestive tract, and the efficient uptake of substances into cells, transferring substances into cells by co-transporting neutral and basic amino acids (see Non-Patent Documents 4 and 5), D-amino acids (Non-Patent Document 6), carnitines (Non-Patent Document 7), and such into cells with two sodium ions and one chloride ion.

There is a PCT application (Patent Document 1) relating to the antigenic ATB^{0,+} gene and protein, and describing ATB^{0,+} as a glycine transporter.

In humans, ATB^{0,+} is expressed in the following organs: Master blotting (mRNA, Clontech): lung, trachea, salivary gland (high), mammary gland, stomach, pituitary gland (low), colon, uterus, testis, and prostate (even lower) (Non-Patent Document 4).

Furthermore, in mice, ATB^{0,+} is expressed in the following organs: Northern blotting: colon (high) and lung (low) (see Non-Patent Documents 5 and 8); Immunostaining: colon (see Non-Patent Document 6), and lung and trachea (all on the luminal side) (reported by Mager, in PharmaConference 2001, Interlaken, Switzerland).

However, it was unclear whether ATB^{0,+} was involved in cancer cell growth. Thus, there had been no discussion on whether or not inhibiting ATB^{0,+} function would influence cancer cell growth.

### [Patent Document 1] WO 2000/14221

[Non-Patent Document 1] Ganapathy, V. *et al.,* In Current Topics in Membranes. Ed. B arrett, K.E. and Donowitz, M., 2001, Vol.50, p.379-412
[Non-Patent Document 2] Osiecka *et al.,* J. Pharmacol. Exp. Ther., 1987, Vol.242, p.443 -449
[Non-Patent Document 3] Hatanaka *et al.,* Pharm. Res., 1999, Vol.16, p.1770-1774
[Non-Patent Document 4] Sloan, J.L., Mager, S., J. Biol. Chem., 1999, Vol.274, p.23740 -23745
[Non-Patent Document 5] Hatanaka *et al.,* J. Clin. Invest. 2001, Vol. 107, p.1035-1043
[Non-Patent Document 6] Hatanaka *et al.,* Biochem. Biophys. Res. Commun., 2002, Vol. 291, p.291-295
[Non-Patent Document 7] Nakanishi *et al.,* J. Physiol., 2001, Vol.532(2), p.297-304
[Non-Patent Document 8] Ugawa *et al.,* Am. J. Physiol., 2001, Vol.281, G365-G370

### Disclosure of the Invention

The present invention was made in view of this situation. An objective of this invention is to provide cell growth inhibitors comprising an amino acid transporter ATB^{0,+} inhibitory substance as an active ingredient, and in particular, cancer cell growth inhibitors for colon cancer or such.

The present inventors vigorously studied to solve the above-mentioned problems. In canine small intestinal brush border membrane with strong ATB^{0,+} activity, L-2-phenylglycine shows a stronger affinity than the similar L-alanine and L-phenylalanine, and is thus considered to be a competitive inhibitor, highly specific to ATB^{0,+} (Hatanaka *et al.* 2002, J. Pharm. Pharmcol.). The present inventors used L-2-phenylglycine, which is considered to be an ATB^{0,+} inhibitor, as a test substance to perform cell growth tests on human colon cancer cell lines SW60 and HT-29, and breast cancer cell line MCF-7. As a result, cell growth suppression due to L-2-phenylglycine was not observed in SW60. On the other hand, cell growth suppression dependent on L-2-phenylglycine concentration was observed in HT-29 and MCF-7. According to RT-PCR results, ATB^{0,+} expression was high in MCF-7 and HT-29, and below detectable limits in SW60. From the above, cell growth suppression by L-2-phenylglycine was suggested to be due to inhibition of ATB^{0,+} function rather than non-specific cytotoxicity.

As described above, the present inventors discovered that cell growth can be suppressed by inhibiting the activity of amino acid transporter ATB^{0,+}, and completed the present invention. The discoveries of the present inventors enabled the development of cell growth inhibitors that use amino acid transporter ATB^{0,+} as a target. More specifically, the present inventors found that the cell growth may be suppressed by inhibiting the activity of the amino acid transporter ATB^{0,+}. Suppression of amino acid transporter ATB^{0,+} activity may be an important index in developing growth inhibitors for cancer cells or such. There is much expectation that the cell growth inhibitors of the present invention will be utilized for suppressing the growth of cancers (such as colon cancer and breast cancer).

The present invention relates to cell growth inhibitors comprising an amino acid transporter ATB^{0,+} inhibitory substance as an active ingredient. More specifically, the present invention provides:
[1] a cell growth inhibitor comprising an amino acid transporter ATB^{0,+} inhibitory substance as an active ingredient;
[2] the cell growth inhibitor of [1], wherein the amino acid transporter is a Na⁺ and Cl⁻-driven amino acid transporter;
[3] the cell growth inhibitor of [1], wherein the amino acid transporter is ATB^{0,+};
[4] the cell growth inhibitor of [1], wherein the amino acid transporter ATB^{0,+} inhibitory substance is a substance that inhibits the transport function of the amino acid transporter ATB^{0,+} by binding to the amino acid transporter ATB^{0,+};
[5] the cell growth inhibitor of [1], wherein the amino acid transporter ATB^{0,+} inhibitory substance is selected from the group consisting of L-amino acids, NOS inhibitors, phenylglycine derivatives, carnitines, D-amino acids, and amino acid-based prodrugs, or the group consisting of derivative compounds thereof;
[6] the cell growth inhibitor of [1], wherein the amino acid transporter ATB^{0,+} inhibitory substance is an antibody that binds to the amino acid transporter ATB^{0,+};
[7] the cell growth inhibitor of [6], wherein the antibody that binds to the amino acid transporter ATB^{0,+} has cytotoxicity;
[8] the cell growth inhibitor of [7], wherein the cytotoxicity is antibody-dependent cell-mediated cytotoxicity (ADCC activity);
[9] the cell growth inhibitor of [7], that is an antibody with complement-dependent cytotoxicity (CDC activity);
[10] the cell growth inhibitor of [1], wherein the amino acid transporter ATB^{0,+} inhibitory substance suppresses the expression of the amino acid transporter ATB^{0,+};
[11] the cell growth inhibitor of any one of [1] to [10], that suppresses growth of a cancer cell; and
[12] the cell growth inhibitor of [11], wherein the cancer cell is a colon cancer cell, pancreatic cancer cell, or breast cancer cell.

The present invention provides cell growth inhibitors comprising an amino acid transporter ATB^{0,+} inhibitory substance as an active ingredient. The amino acid transporter ATB^{0,+} targeted by the cell growth inhibitors of this invention is a Na⁺ and Cl⁻-driven amino acid transporter. The amino acid transporter ATB^{0,+} transports neutral or basic amino acids (Sloan *et al.,* J. Biol. Chem., 274, p23740-23745, 1999; Hatanaka *et al.,* J. Clin. Invest., 107, p1035-1043, 2001), D-amino acids (Hatanaka *et al.,* Biochem. Biophys. Res. Commun., 291, p291-295, 2002), carnitines (Nakanishi *et al.,* J. Physiol., 532, 2, p297-304, 2001), and such into cells by cotransport with two sodium ions and one chloride ion, and is considered to be involved in efficient absorption of nutritional substances in the digestive tract, and efficient uptake of substances into cells.

The amino acid sequence of the amino acid transporter ATB^{0,+} protein and the nucleotide sequence of the gene encoding this protein are already known (GenBank Accession No. 151978).

The amino acid transporter ATB^{0,+} inhibitory substances of this invention are not particularly limited, as long as they suppress cell growth by inhibiting transport mediated by the amino acid transporter ATB^{0,+}, or by binding to the amino acid transporter ATB^{0,+} to cause cytotoxicity. Substances that inhibit transport mediated by the amino acid transporter ATB^{0,+} include, for example, substances that bind to the amino acid transporter ATB^{0,+} to inhibit its transport function, and substances that suppress its expression. Substances that bind to the amino acid transporter ATB^{0,+} to inhibit its transport function are preferred.

Examples of the amino acid transporter ATB^{0,+} inhibitory substances of this invention include synthetic low-molecular-weight compounds, antibodies, proteins, peptides, and naturally occurring compounds. More specifically, these include NOS inhibitors, phenylglycine, carnitines, D-amino acids, and amino acid-based prodrugs, and derivatives thereof.

NOS inhibitors of this invention are preferably those with neutral or basic L-α-amino acid-based structures, and more preferably those with L-arginine, L-lysine, L-citrulline, or L-ornithine-based structures. Even more preferably are arginine, lysine, citrulline, ornithine, L-NNA (N^{G}-nitro-L-arginine), L-NAME (N^{G}-nitro-L-arginine methyl ester), L-NMMHA (N^{G}-monomethyl-L-homoarginine), L-NDMA (N^{G}N^{G'}-dimethyl-L-arginine), L-NMEA (N^{G}-monoethyl-L-arginine), L-NMMA (N^{G}-monomethyl-L-arginine), L-NABE (N^{G}-L-nitro-L-arginine benzyl ester), L-NIL (L-N⁶-(1-iminoethyl)-lysine), L-TC (L-thiocitrulline), L-MTC (S-methyl-L-thiocitrulline), L-NIO (L-N⁵-(1-iminoethyl)-ornithine), GGA (α-guanidinoglutaric acid), or canavanine.

The phenylglycines or derivatives thereof of this invention are typically drugs or prodrugs with a phenylglycine structure, preferably phenylglycine derivatives (which are not acidic compounds under physiological pH) comprising free α-amino acids and α-carboxyl groups, or 3- or 4-carboxylester prodrugs comprising 3- or 4-carboxyphenylglycine, and more preferably L-2-phenylglycine.

The carnitines or derivatives thereof of this invention are preferably carnitines or acyl esters thereof, and more preferably carnitine, acetylcarnitine, or propionylcarnitine.

The D-amino acids of this invention are preferably D-alanine, D-serine, D-methionine, D-leucine, D-tryptophan, D-threonine, D-histidine, D-phenylalanine, D-glutamine, or derivatives thereof, more preferably D-alanine, D-serine, D-methionine, D-leucine, D-tryptophan, or derivatives thereof, and even more preferably D-alanine, D-serine, D-methionine, D-leucine, or D-tryptophan.

The amino acid-based prodrugs of this invention are preferably prodrugs comprising an amino acid functional group (including D- or L-amino acids), more preferably β-carboxylester prodrugs or γ-carboxylester prodrugs comprising an aspartic acid or glutamic acid, and α-carboxylester prodrugs comprising a neutral or basic amino acid.

In the present invention, the above-mentioned compounds can be appropriately labeled, as necessary. The labels may be, for example, radiolabels and fluorescent labels.

In a preferred embodiment of the present invention, the amino acid transporter ATB^{0,+} inhibitory substances are antibodies against the amino acid transporter ATB^{0,+} protein. The antibodies typically recognize and bind to the ATB^{0,+} protein.

The type of antibody comprised in the cell growth inhibitors of this invention is not particularly limited, so long as it can bind to the amino acid transporter ATB^{0,+}. Antibodies that bind specifically to the amino acid transporter ATB^{0,+} are preferable; and cytotoxic antibodies are more preferable.

Cytotoxicity in this invention includes antibody-dependent cell-mediated cytotoxicity (ADCC) and complement-dependent cytotoxicity (CDC). In the present invention, CDC activity means cytotoxicity mediated by a complement system. ADCC activity in the present invention means an activity cytotoxic to a target cell, where a specific antibody binds to a surface antigen of the target cell, and the Fc moiety binds an Fcγ receptor-containing cell (such as an immunocyte) via the Fcγ receptor.

Whether an anti-amino acid transporter ATB^{0,+} antibody has either ADCC activity or CDC activity can be determined by methods well known in the art (for example, Current Protocols in Immunology, Chapter 7. Immunologic studies in humans, Editor, John E. Coligan *et al.,* John Wiley & Sons, Inc. (1993)).

Specifically, effector cells, complement solution, and target cells are first prepared.

### (1) Preparation of effector cells

Spleen is excised from a CBA/N mouse or such, and spleen cells are isolated in RPMI1640 medium (GIBCO). After washing cells with the same medium containing 10% fetal bovine serum (FBS) (HyClone), cell density is adjusted to 5x 10⁶ cells/ml, preparing the effector cells.

### (2) Preparation of complement solution

Baby Rabbit Complement (CEDARLANE) is diluted 10-fold in a medium containing 10% FBS (GIBCO), preparing the complement solution.

### (3) Preparation of target cells

Pancreatic cancer cell line (*e.g.*, AsPc-1 or Capan-2) cells are radiolabeled by incubation with 0.2 mCi ⁵¹Cr-sodium chromate (Amersham Pharmacia Biotech) in DMEM medium containing 10% FBS at 37°C for one hour. Then, cells are washed three times with RPMI1640 medium containing 10% FBS, and adjusted to a cell density of 2x 10⁵ cells/ml, preparing the target cells.

ADCC or CDC activity is then measured. When measuring ADCC activity, the target cells and anti-amino acid transporter ATB^{0,+} antibodies are added (50 µl each/well) into a 96-well U-bottomed plate (Beckton Dickinson), and reacted on ice for 15 minutes. After the reaction, 100 µl of effector cells are added to each well, and the plate is cultured in a carbon dioxide gas incubator for four hours. The final antibody concentration is made 0 µg or 10 µg/ml. After incubation, the supernatant (100 µl) is collected and radioactivity is measured using a gamma counter (COBRAIIAUTO-GMMA, MODEL D5005, Packard Instrument Company). Cytotoxic activity (%) can be calculated by the formula:

(A-C) / (B-C) x 100

wherein A represents the radioactivity (cpm) of each sample; B represents the radioactivity of a sample comprising 1% NP-40 (Nacalai); and C represents the radioactivity of a sample comprising only the target cells.

When measuring CDC activity, the target cells and anti-amino acid transporter ATB^{0,+} antibodies are added (50 µl each/well) to a 96-well flat-bottomed plate (Becton Dickinson), and reacted on ice for 15 minutes. Then, the complement solution (100 µl) is added to each well, and cultured in a carbon dioxide gas incubator for four hours. The final antibody concentration is made 0 µg or 3 µg/ml. After incubation, the supernatant (100 µl) is recovered and its radioactivity measured using a gamma counter. Cytotoxicity can be calculated as for the ADCC activity assay.

The antibodies used as the active ingredient of the cell growth inhibitors of the present invention are not particularly limited, as long as they bind an antigen (the amino acid transporter ATB^{0,+} protein or a partial peptide thereof). Mouse antibodies, rat antibodies, rabbit antibodies, sheep antibodies, chimeric antibodies, humanized antibodies, and human antibodies may be appropriately used. Although the antibodies may be either polyclonal or monoclonal antibodies, monoclonal antibodies are preferred since they can stably produce homogeneous antibodies. Polyclonal and monoclonal antibodies can be prepared by methods well known to those skilled in the art.

Basically, hybridoma cells that produce monoclonal antibodies can be produced using conventional techniques, as described below. Specifically, hybridoma cells can be prepared by using normal immunization methods to immunize using a desired antigen, or cells expressing the desired antigen, as the sensitizing antigen; fusing the obtained immunized cells with conventional parent cells by normal cell fusion methods; and screening for monoclonal antibody-producing cells (hybridomas) using normal screening methods.

Antigens can be prepared according to conventional methods, such as methods using baculoviruses (*e.g.* WO 98/46777).

Hybridomas can be produced, for example, according to the method of Milstein *et al.* (Kohler, G and Milstein, C., Methods Enzymol. (1981) 73: 3-46). When an antigen has low immunogenicity, immunization can be performed by linking it to a macromolecule that has immunogenicity, such as albumin.

Recombinant antibodies can also be used, and can be produced by cloning an antibody gene from a hybridoma; incorporating the antibody gene into an appropriate vector; introducing the vector into a host; and producing the recombinant antibodies by genetic engineering techniques (see, for example, Carl, A. K. Borrebaeck, James, W. Larrick, THERAPEUTIC MONOCLONAL ANTIBODIES, Published in the United Kingdom by MACMILLAN PUBLISHERS LTD, 1990). Specifically, the cDNAs of antibody variable regions (V regions) are synthesized from hybridoma mRNAs using reverse transcriptase. When a DNA encoding a V region of an antibody of interest is obtained, it is linked to a DNA encoding an antibody constant region (C region) of interest, and then incorporated into an expression vector. Alternatively, DNAs encoding an antibody V region can be incorporated into expression vectors comprising DNAs of an antibody C region. The DNAs are incorporated into expression vectors so that expression is controlled by expression regulatory regions such as enhancers and promoters. Host cells are then transformed with these expression vectors to express the antibodies.

In the present invention, recombinant antibodies artificially modified to reduce heterologous antigenicity against humans can be used. Examples of such antibodies include chimeric antibodies and humanized antibodies. These modified antibodies can be produced using known methods. A chimeric antibody is an antibody comprising the antibody heavy and light chain variable regions of a nonhuman mammal such as a mouse, and the antibody heavy and light chain constant regions of a human. A chimeric antibody can be obtained by ligating a DNA encoding variable regions of a mouse antibody to a DNA encoding constant regions of a human antibody; incorporating this into an expression vector; and introducing the vector into a host for production of the antibody.

A humanized antibody, which is also called a reshaped human antibody, is obtained by transplanting a complementarity determining region (CDR) of an antibody of a nonhuman mammal such as a mouse, into the CDR of a human antibody. Conventional genetic recombination techniques for the preparation of such antibodies are known. Specifically, a DNA sequence designed to ligate a mouse antibody CDR with a human antibody framework region (FR) is synthesized by PCR, using several oligonucleotides constructed to comprise overlapping portions at their ends. A humanized antibody can be obtained by ligating the resulting DNA to a DNA which encodes a human antibody constant region; incorporating this into an expression vector; and transfecting the vector into a host to produce the antibody (see, European Patent Application No. EP 239,400, and International Patent Application No. WO 96/02576). Human antibody FRs that are ligated via the CDR are selected if the CDR forms a favorable antigen-binding site. Amino acids in the framework region of an antibody variable region may be substituted as necessary, such that the CDR of a reshaped human antibody forms an appropriate antigen-binding site (Sato, K*. et al.,* Cancer Res. (1993) 53, 851-856).

Methods for obtaining human antibodies are also known. For example, desired human antibodies with antigen-binding activity can be obtained by sensitizing human lymphocytes with antigens of interest or cells expressing antigens of interest *in vitro;* and fusing the sensitized lymphocytes with human myeloma cells such as U266 (see Examined Published Japanese Patent Application No. (JP-B) Hei 1-59878). Alternatively, desired human antibodies can also be obtained by using desired antigens to immunize transgenic animals comprising the entire repertoire of human antibody genes (see International Patent Application WO 93/12227, WO 92/03918, WO 94/02602, WO 94/25585, WO 96/34096, and WO 96/33735). Further, techniques to obtain human antibodies by panning with a human antibody library are known. For example, phage display methods are used to express the variable regions of a human antibody as a single chain antibody (scFv) on the surface of a phage, and phages that bind the antigen can be selected. The DNA sequences encoding the variable regions of human antibodies that bind the antigen can be determined by analyzing the genes of selected phages. If the DNA sequences of scFvs that bind the antigen are identified, appropriate expression vectors containing these sequences can be constructed, and human antibodies can be obtained. Such methods are already well known (see WO 92/01047, WO 92/20791, WO 93/06213, WO 93/11236, WO 93/19172, WO 95/01438, and WO 95/15388).

When the antibody genes have been isolated and introduced into an appropriate host, appropriate combinations of hosts and expression vectors can be used to produce the antibodies. Animal cells, plant cells, and fungal cells may be used as eukaryotic host cells. Known animal cells include (1) mammalian cells such as CHO, COS, myeloma, baby hamster kidney (BHK), HeLa, and Vero cells; (2) amphibian cells such as Xenopus oocytes; or (3) insect cells such as sf9, sf21, and Tn5. Known plant cells include cells derived from the *Nicotiana* genus such as *Nicotiana tabacum,* which can be callus cultured. Known fungal cells include yeasts such as the *Saccharomyces* genus, for example *Saccharomyces cerevisiae,* and filamentous fungi such as the *Aspergillus* genus, for example *Aspergillus niger.* Prokaryotic cells can also be used in production systems that utilize bacterial cells. Known bacterial cells include *E*. *coli* and *Bacillus subtilis.* The antibodies can be obtained by transferring the antibody genes of interest into these cells using transformation, and then culturing the transformed cells *in vitro.*

Furthermore, the antibodies of this invention may be antibody mutants. In the present invention, antibody mutants refer to amino acid sequence variants of the antibodies, in which one or more amino acid residues have been modified. The "antibody mutants" of this invention include any modified amino acid variant, as long as it has the same binding specificity as that of the original antibody. Such mutants have sequence homology or similarity of less than 100% to an amino acid sequence with at least 75%, more preferably at least 80%, more preferably at least 85%, more preferably 90%, and most preferably at least 95% amino acid sequence homology or similarity to the amino acid sequence of the variable domain of a heavy or light chain of the antibody.

Furthermore, the above-described antibodies of the present invention may be antibody fragments or modified antibodies thereof, as long as they bind to the amino acid transporter ATB^{0,+} protein and inhibit its function. For example, the antibody fragment may be Fab, F(ab')2, Fv, or single chain Fv (scFv) in which Fv from H or L chains are ligated by an appropriate linker. More specifically, a gene encoding Fab, F(ab')2, Fv, or single chain Fv (scFv) in which Fv's from an H or L chain are ligated, is inserted into an expression vector; and then expressed in an appropriate host cell (see, for example, Co, M. S. *et al.,* J. Immunol. (1994) 152, 2968-2976; Better, M. & Horwitz, A. H. Methods in Enzymology (1989) 178, 476-496, Academic Press, Inc.; Plueckthun, A. & Skerra, A. Methods in Enzymology (1989) 178, 476-496, Academic Press, Inc.; Lamoyi, E., Methods in Enzymology (1989) 121, 663-669; and Bird, R. E. *et al.,* TIBTECH (1991) 9, 132-137). scFv's can be obtained by ligating the V regions of an antibody H-chain and L-chain. In the scFv's, the V regions of the H chain and L chain are ligated via a linker, and preferably via a peptide linker (Huston, J. S. *et al.,* Proc. Natl. Acad. Sci. U.S.A (1988) 85, 5879-5883). The V regions of the scFv H and L chain may be derived from any of the antibodies described herein. The peptide linker used to ligate the V regions may be, for example, any single-chain peptide consisting of 12 to 19 residues. DNAs encoding the scFv's can be amplified by PCR, using either a whole DNA or a partial DNA encoding a desired amino acid sequence as a template, where this is selected from DNAs encoding an H chain or V region of an H chain of the above antibody, and DNAs encoding an L chain or V region of an L chain of the above antibody; and using a primer pair that defines the two ends. Further amplification can be subsequently conducted by combining the DNA encoding the peptide linker portion, and the primer pair that defines both ends of the DNAs to be ligated to the H chain and L chain respectively. Once DNAs encoding scFv's are constructed, expression vectors containing the DNAs, and hosts transformed by these expression vectors, can be obtained by conventional methods. Furthermore, scFvs can be obtained by conventional methods using the resulting hosts. These antibody fragments can be produced in hosts by obtaining genes encoding the antibody fragments and expressing them in a manner similar to that outlined above. Antibodies bound to various types of molecules, such as polyethyleneglycol (PEG), may be used as modified antibodies. Such modified antibodies can be obtained by chemical modification of the resulting antibodies. Methods for modifying antibodies are already established in the art. The term "antibody" in the present invention also encompasses the above-described antibodies.

Furthermore, the antibodies used in the present invention may be bispecific antibodies. The bispecific antibodies may have antigen-binding sites recognizing different epitopes on the amino acid transporter ATB^{0,+} molecule, or may have one antigen-binding site recognizing the amino acid transporter ATB^{0,+} and the other recognizing a cytotoxic substance such as a radioactive substance, chemotherapeutic agent, and cell-derived toxin. In this case, tumor cell growth can be inhibited by directly applying the cytotoxic substance to cells expressing the amino acid transporter ATB^{0,+} to specifically damage them. Bispecific antibodies can be prepared by linking HL pairs of two kinds of antibodies, or obtained by fusing hybridomas that produce different monoclonal antibodies to prepare fused cells generating bispecific antibodies. Further, the bispecific antibodies can be generated using genetic engineering techniques.

Antibodies expressed and produced as described above can be purified by conventional methods for purifying normal proteins. Antibodies can be separated and purified by, for example, appropriately selecting and/or combining affinity columns such as a protein A column, or by chromatography column, filtration, ultrafiltration, salt precipitation, dialysis, and such (Antibodies A Laboratory Manual. Ed Harlow, David Lane, Cold Spring Harbor Laboratory, 1988).

Conventional means can be used to measure the antigen-binding activity of the antibodies (Antibodies A Laboratory Manual. Ed Harlow, David Lane, Cold Spring Harbor Laboratory, 1988). For example, enzyme linked immunosorbent assays (ELISA), enzyme immunoassays (EIA), radioimmunoassays (RIA), or fluoroimmunoassays may be used.

Those skilled in the art can use conventional methods to determine whether a particular molecule binds to the amino acid transporter ATB^{0,+} of the present invention. Examples of conventional methods include immunoprecipitation, West-Western blotting, ELISA, EIA, RIA, fluoroimmunoassay, and methods using a biosensor using surface plasmon resonance effect.

Candidate compounds for the cell growth inhibitors of the present invention can be screened using the activity of binding to the amino acid transporter ATB^{0,+} as an indicator. Specifically, a test sample is contacted with the amino acid transporter ATB^{0,+}, binding between the amino acid transporter ATB^{0,+} and a test sample is detected, and compounds that bind to the amino acid transporter ATB^{0,+} can be selected. There are no particular limitations as to the test samples. Examples include cell extracts, cell culture supernatants, products of fermenting microorganisms, extracts from marine organisms, plant extracts, purified or crude purified proteins (including antibodies), peptides, non-peptidic compounds, synthetic low-molecular-weight compounds, and natural compounds. The amino acid transporter can be contacted with test samples as a purified protein, in a form bound to a carrier, as a fusion protein with another protein, in a form expressed on a cell membrane, or as a membrane fraction, for example. When selecting strong candidates for the cell growth inhibitors of this invention from the compounds that bind amino acid transporter ATB^{0,+} obtained in this manner, it is useful to determine whether these compounds inhibit the transport function of amino acid transporter ATB^{0,+}. Whether a particular molecule inhibits the transport function of a peptide transporter can be judged by conventional methods, for example, by labeling a substrate such as an amino acid with a radioactive (*e.g*. ¹⁴C) or fluorescent substance, and then measuring the amount of substrate incorporated into cells expressing the amino acid transporter.

Substances that suppress the expression of the amino acid transporter ATB^{0,+} can be used as active ingredients for the cell growth inhibitors of this invention. Examples of such substances include antisense oligonucleotides against the amino acid transporter ATB^{0,+} gene. Examples of antisense oligonucleotides include an antisense oligonucleotide that hybridizes to any site of a DNA or mRNA encoding the amino acid transporter ATB^{0,+}. Preferably, the antisense oligonucleotide is against at least 15 or more continuous nucleotides in the DNA or mRNA of the amino acid transporter. More preferably, the antisense oligonucleotide is against at least 15 or more continuous nucleotides comprising a translation initiation codon. Derivatives and modified forms of these can also be used as antisense oligonucleotides, including modified lower alkylphosphonates such as methyl phosphonate forms and ethyl phosphonate forms, modified phosphorothioate, or modified phosphoroamidate.

The cells to be targeted by the cell growth inhibitors of the present invention are not particularly limited, but cancer cells such as colon cancer cells, breast cancer cells, and pancreatic cancer cells are preferred. Colon cancer cells and breast cancer cells are especially preferred.

The cell growth inhibitors of the present invention are used to treat and prevent diseases caused by cell growth, and more specifically to treat and prevent cancers such as colon cancer or breast cancer.

As mentioned above, the amino acid transporter ATB^{0,+} is not expressed in the vascular-side cell membrane. Accordingly, the amino acid transporter ATB^{0,+} inhibitory substances (for example, antibodies that bind to the amino acid transporter ATB^{0,+} are considered to have specific activities on cancer cells of colon cancer or such, without inhibiting the function of the amino acid transporter ATB^{0,+} in normal tissues. Furthermore, by inference from physiological functions, amino acid transporter ATB^{0,+} abnormalities in colon cancer cells or such may be involved in enhanced amino acid uptake, which is necessary for the protein synthesis that accompanies cancer cell growth. Therefore, the cell growth inhibitors of this invention may be novel anticancer agents, which have the pharmaceutical effect of nutritional obliteration by the amino acid transporter ATB^{0,+} inhibitory substance. Furthermore, in a preferable embodiment of the present invention, the cell growth inhibitors, which comprise antibodies that bind to the amino acid transporter ATB^{0,+} as an active ingredient, are expected to be novel anticancer agents with the pharmaceutical effect of killing cells. This is caused by the antibody binding to ATB^{0,+} to activate cells that are cytotoxic against tumor cells (antibody-dependent cell-mediated cytotoxicity; ADCC activity), or cytocidal effects by a complement protein (complement-dependent cytotoxicity; CDC activity).

To formulate the pharmaceutical agents of this invention, pharmaceutically acceptable carriers can be added as necessary, according to conventional methods. Exemplary carriers include, but are not limited to, surfactants, excipients, coloring agents, flavoring agents, preservatives, stabilizers, buffers, suspensions, isotonizing agents, binding agents, disintegrators, lubricants, fluidity promoters, and corrigents, and other appropriate ordinary carriers. More specific examples include light anhydrous silicic acid, lactose, crystalline cellulose, mannitol, starch, carmellose calcium, carmellose sodium, hydroxypropylcellulose, hydroxypropylmethylcellulose, polyvinylacetal diethylaminoacetate, polyvinylpyrrolidone, gellatin, medium chain fatty acid triglyceride, polyoxyethylene hydrogenated castor oil 60, sucrose, carboxymethylcellulose, cornstarch, and inorganic salts.

The dosage forms of the above-mentioned pharmaceutical agents include tablets, epipastics, pills, powders, granules, fine granules, soft/hard capsules, film-coated agents, pellets, buccals, and pastes as oral agents; and injections, suppositories, percutaneous agents, ointments, plasters, and liquid for external use as parenteral agents. Those skilled in the art can select the most appropriate dosage form depending on the administration route, administration target, and such.

The cell growth inhibitors of the present invention can be administered either orally or parenterally, but are preferably administered parenterally. Specific examples include injections, nasal formulations, pulmonary formulations, and cutaneous formulations. For example, injections can be administered systemically or locally by intravenous injection, intramuscular injection, intraperitoneal injection, or subcutaneous injection. Furthermore, the method of administration can be appropriately selected according to the age and symptoms of the patient. A single dose can be selected, for example, from within the range of 0.0001 mg to 1,000 mg per kg of body weight. Alternatively, doses can be selected, for example, from within the range of 0.001 to 100,000 mg/body for each patient. However, the dose of a therapeutic agent of the present invention is not limited to these examples. Furthermore, the therapeutic agents of the present invention can be formulated according to standard methods (see, for example, Remington's Pharmaceutical Science, latest edition, Mark Publishing Company, Easton, U.S.A), and may comprise pharmaceutically acceptable carriers and additives.

### Brief Description of the Drawings

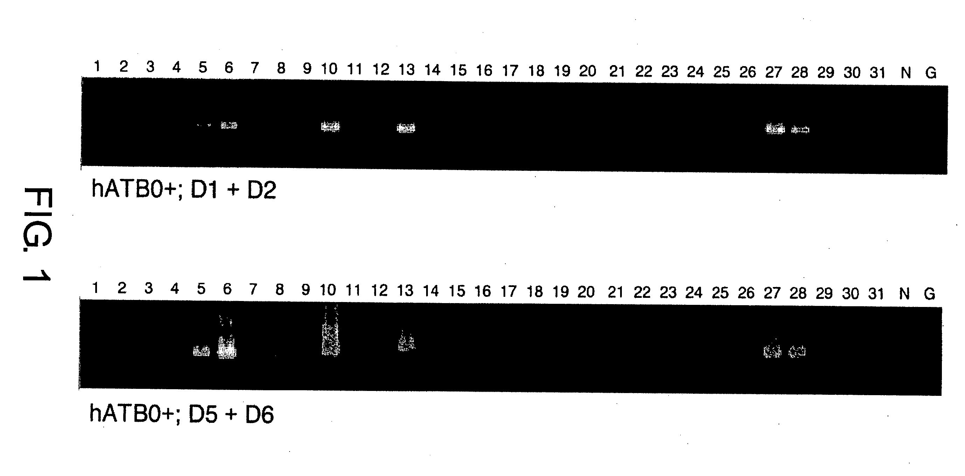

Fig. 1 is a set of photographs showing expression of the amino acid transporter ATB^{0,+} in each type of cancer cell line. The photograph on the left was taken when PCR was performed using a combination of D1 and D2 primers; and the photograph on the right was taken when PCR was performed using a combination of D5 and D6 primers. Well numbers correspond to the following cell lines (Table 1):

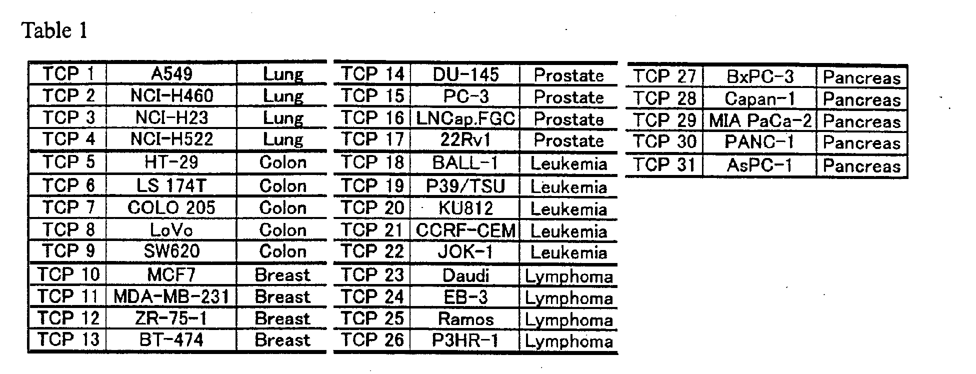

TCP1 and TCP2 correspond to well 1 and well 2, respectively. "N" refers to a case when no template DNA was added; and "G" refers to a case when human chromosomal DNAs were added.

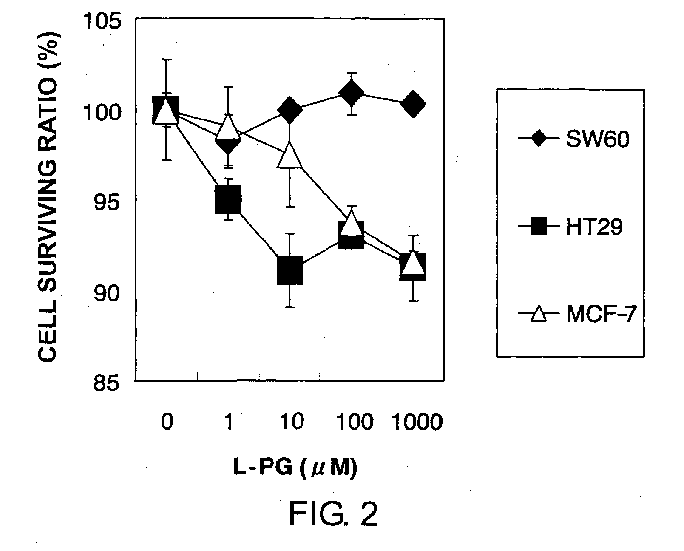

Fig. 2 shows the suppression effect of L-2-phenylglycine on each type of cancer cell line.

### Best Mode for Carrying Out the Invention

Herein below, the present invention will be specifically described using Examples, but it is not to be construed as being limited thereto.

### [Example 1] RT-PCR analysis of human ATB^{0,+}

### (1-1) Preparation of total RNAs

From the following human cancer cell lines, total RNAs were prepared using ISOGEN (Nippon Gene) or TRIzo1 (Invitrogen), according to standard methods recommended by their manufacturers.
- Lung cancer cell lines: A549, NCI-H460, NCI-H23, NCI-H522
- Colon cancer: HT-29, LS 174T, COLO 205, LoVo, SW620
- Breast cancer cell lines: MCF7, MDA-MB-231, ZR-75-1, BT-474
- Prostate cancer cell lines: DU-145, PC-3, LNCap.FGC, 22Rv1
- Leukemia cell lines: BALL-1, P39/TSU, KU812, CCRF-CEM, JOK-1
- Lymphoma cell lines: Daudi, EB-3, Ramos, P3HR-1
- Pancreatic cancer cell lines: BxPC-3, Capan-1, MIA PaCa-2, PANC-1, AsPC-1

### (1-2) RT-PCR analysis

Single-stranded cDNAs were synthesized from the above total RNAs using SUPERSCRIPT^{TM} First-Strand Synthesis System for RT-PCR (Invitrogen), according to standard methods recommended by the manufacturer. Oligo dTs (12 to 18 mer) were used as primers for a reverse transcription reaction. Using a portion of the obtained cDNAs as the template, PCR analysis was performed with primers specific to the human ATB^{0,+} gene under the following conditions:

### <Reaction solution composition>

| | |
|---|---|
| TaKaRa ExTaq (TaKaRa) | 0.3 µL |
| TaqStart^{TM} Antibody (CLONTECH) | 0.3 µL |
| 10x ExTaq buffer (TaKaRa) | 3 µL |
| 2.5 mM dNTPs (TaKaRa) | 2.4 µL |
| 20 µM primers | each 0.6 µL |
| Template cDNA | 3 µL |
| Total | 30 µL |

### <Reaction conditions>

94°C for two minutes
→ (94°C for 30 seconds → 68°C for two minutes) x 35 cycles
→ 72°C for five minutes

### <Primers>

D1: 5'- cag ttt cag ttt gga gga ttc ttc -3' (SEQ ID NO: 1)

D2: 5'- tta ctc cag tct cat tca ttc cac -3' (SEQ ID NO: 2)

D5: 5'- ctg ctt ggt ttt gtt tct cct tgg -3' (SEQ ID NO: 3)

D6: 5'- aat cat aca cca gcc taa agc aac -3' (SEQ ID NO: 4)

The results of PCR are shown in Fig. 1. Of the five colon cancer cell lines (lanes 5 to 9), two cell lines (HT-29 and LS 174T) showed strong expression. Medium level expression was observed in Lovo cells. All of the four breast cancer cell lines (lanes 10 to 13) showed ATB^{0,+} expression, and MCF7 and BT-474 showed particularly strong expression. Of the five pancreatic cancer cell lines (lanes 27 to 31), two cell lines (BxPC-3 and Capan-1) showed strong expression. Although heterogeneous cancers were used, ATB^{0,+} was highly expressed in 40% or more of the cell lines in colon cancer, breast cancer, and pancreatic cancer, as shown in Fig. 1. Clinically it was also suggested that ATB^{0,+} can be highly expressed at the tumor sites of 40% or more of patients with colon cancer, breast cancer, and pancreatic cancer.

### [Example 2] The cell growth suppression effect of L-2-phenylglycine on human colon cancer cell lines SW60 and HT29, and breast cancer cell line MCF-7

L-2-phenylglycine shows a stronger affinity than the similar L-alanine and L-phenylalanine in canine small intestinal brush border membrane that shows strong ATB^{0,+} activity. Accordingly, L-2-phenylglycine is considered to be a competitive inhibitor with strong specificity to ATB^{0,+} (Hatanaka *et al.* 2002, J. Pharm. Pharmacol.). The present inventors prepared 20 mM L-2-phenylglycine solution by dissolving L-2-phenylglycine in a medium containing 0% FBS. IMDM was used as the medium for SW60 and HT29; and RPMI was used for MCF-7. This solution was diluted with the medium to prepare L-2-phenylglycine solutions of 2, 0.2, 0.02, and 0.002 mM.

Human colon cancer cell lines SW60 and HT29, and breast cancer cell line MCF-7 were prepared with medium to form 4x 10⁶, 1.6x 10⁶, and 1.6x 10⁶ cells/mL, respectively. 25 µL/well of the suspensions thus obtained (1x10⁵, 4x 10⁴, and 4x 10⁴ cells, respectively) were plated onto a 96-well plate, which already contained 25 µL/well of medium comprising 12% FBS. 50 µL of L-2-phenylglycine solution was then added to each well. The cells were cultured in a 5% CO₂ incubator, and surviving cell count was quantified by MTS assay on the fourth day of culturing.

Fig. 2 shows the results of examining cell growth. Cell growth suppression due to L-2-phenylglycine could not be observed in SW60. On the other hand, concentration-dependent cell growth suppression was observed in HT29 and MCF-7. In the presence of 10 µM L-2-phenylglycine, cell growth suppression in HT29 was approximately 10% and reached equilibrium. Cell growth suppression in MCF-7 was approximately 10% in the presence of 1 mM L-2-phenylglycine.

According to the RT-PCR results, ATB^{0,+} expression levels were high in MCF-7 and HT29, and below the detection limit in SW60 (Fig. 1). Accordingly, cell growth suppression by L-2-phenylglycine was considered to be due to the inhibition of ATB^{0,+} function, rather than to a non-specific cytotoxicity.

### Industrial Applicability

The present invention revealed that substances inhibiting the activity of the amino acid transporter ATB^{0,+} suppress cell growth effectively. Accordingly, cell growth inhibitors can be developed using the amino acid transporter ATB^{0,+} as a target. Such cell growth inhibitors are highly expected to be of use in suppressing the growth of cancers (for example, colon cancer).

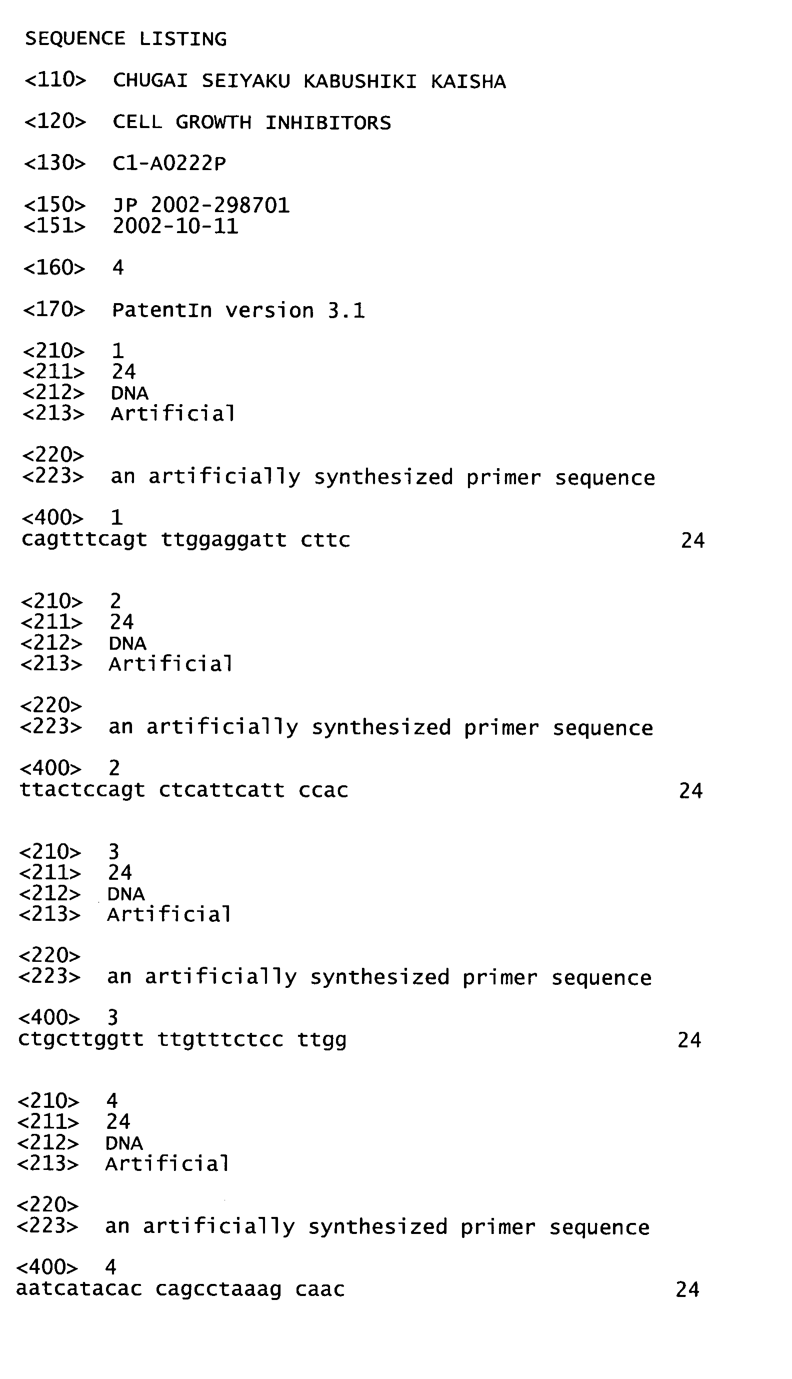

## Claims

1. A cell growth inhibitor comprising an amino acid transporter ATB^{0,+} inhibitory substance as an active ingredient.

2. The cell growth inhibitor of claim 1, wherein the amino acid transporter is a Na⁺ and Cl⁻-driven amino acid transporter.

3. The cell growth inhibitor of claim 1, wherein the amino acid transporter is ATB^{0,+}.

4. The cell growth inhibitor of claim 1, wherein the amino acid transporter ATB^{0,+} inhibitory substance is a substance that inhibits the transport function of the amino acid transporter ATB^{0,+} by binding to the amino acid transporter ATB^{0,+}.

5. The cell growth inhibitor of claim 1, wherein the amino acid transporter ATB^{0,+} inhibitory substance is selected from the group consisting of L-amino acids, NOS inhibitors, phenylglycine derivatives, carnitines, D-amino acids, and amino acid-based prodrugs, or the group consisting of derivative compounds thereof.

6. The cell growth inhibitor of claim 1, wherein the amino acid transporter ATB^{0,+} inhibitory substance is an antibody that binds to the amino acid transporter ATB^{0,+}.

7. The cell growth inhibitor of claim 6, wherein the antibody that binds to the amino acid transporter ATB^{0,+} has cytotoxicity.

8. The cell growth inhibitor of claim 7, wherein the cytotoxicity is antibody-dependent cell-mediated cytotoxicity (ADCC activity).

9. The cell growth inhibitor of claim 7 that is an antibody with complement-dependent cytotoxicity (CDC activity).

10. The cell growth inhibitor of claim 1, wherein the amino acid transporter ATB^{0,+} inhibitory substance suppresses the expression of the amino acid transporter ATB^{0,+}.

11. The cell growth inhibitor of any one of claims 1 to 10 that suppresses growth of a cancer cell.

12. The cell growth inhibitor of claim 11, wherein the cancer cell is a colon cancer cell, pancreatic cancer cell, or breast cancer cell.
